Europäisches Patentamt

European Patent Office

Office européen des brevets

(11) Publication number: **0 414 541 A1**

## EUROPEAN PATENT APPLICATION

(21) Application number: 90309253.4

(22) Date of filing: 23.08.90

(51) Int. Cl.⁵: **D04H 1/70**, D04H 1/42, A61F 13/15

(30) Priority: 25.08.89 US 389209

(43) Date of publication of application:
27.02.91 Bulletin 91/09

(84) Designated Contracting States:
**BE DE ES FR GB IT NL**

(71) Applicant: ARCO CHEMICAL TECHNOLOGY INC.
3 Christina Centre Suite 902 201 N Walnut Street
Wilmington Delaware 19801(US)

(72) Inventor: Gupta, Vijai P.
816 Newton Road
Berwyn, Pennsylvania 19312(US)

(74) Representative: Cropp, John Anthony David et al
MATHYS & SQUIRE 10 Fleet Street
London, EC4Y 1AY(GB)

(54) Fiber structures with absorbency gradient and process for their production.

(57) Superabsorbent non-wovens prepared by air-laying superabsorbent staple fibers along with other fibers, differing in density by at least 0.2 g/cc from that of the superabsorbent fiber.

# FIBER STRUCTURES WITH ABSORBENCY GRADIENT AND PROCESS FOR THEIR PRODUCTION

This invention relates to multi-component superabsorbent articles exhibiting both high strength and excellent liquid absorption properties. The multi-component articles of this invention are air-laid non-woven mats of highly absorbent fibers and at least one other type of fiber. The webs are useful per se or may be combined with other materials to form a variety of structures, such as diapers, sanitary napkins, bandages, incontinency products, and others for the absorption of water and electrolytic solutions, such as body fluids.

## Information Disclosure Statement

U.S. 2,500,282 discloses felt-like fibrous products comprising mixtures of at least two types of fibers. The fibrous webs or mats of this invention are obtained by assembling and superimposing upon one another a multitude of relatively thin webs or layers. One of the fibers is a heat-activatible fiber, which fiber after exposure to heat holds the various layers together.

U.S. 3,016,599 discloses a method for producing homogeneous mixtures of staple fibers in which one of the fibers is considerably smaller than the other fibers.

U.S. 4,100,324 discloses non-woven fabrics comprised of wood pulp fibers and polymeric fibers in which the wood pulp fibers and the synthetic fibers are intimately integrated. The non-woven web is formed by an air-laying process. One advantage of the invention is said to be the homogeneous distribution of both fibrous materials throughout the final composite web.

U.S. 4,375,447 discloses a method for forming an air-laid web with dry fibers.

U.S. 4,604,313 discloses a layered non-woven fabric-like material comprised of an absorbent layer of synthetic staple fibers having microfibers dispersed throughout and a second absorbent layer having superabsorbent particles bonded to the first absorbent layer. A composite web is thus formed in which the absorbency of the layers differs.

U.S. 4,731,067 discloses a water-absorbing composition useful in this invention.

## Detailed Description of the Drawings

Fig. 1 is a micrograph of the front and back of a non-woven web of this invention showing the distribution of superabsorbent and polypropylene fibers. The superabsorbent fibers appear as the rod-like structures embedded in the spaghetti-like polypropylene fibers.

Fig. 2 is a plot of absorbency versus time for the 50-50 blends of superabsorbent fiber with polyester and polypropylene fibers.

## Summary of the Invention

According to the present invention, it has been found that superabsorbent non-woven webs may be prepared by air-laying superabsorbent staple fibers along with other synthetic fibers, which have a density differing by at least 0.2 g/cc from that of the superabsorbent fiber. As used herein, the term "superabsorbent" refers to fibers which will absorb from about 20 to about 300 times their weight of liquids.

This invention provides a non-woven web comprised of at least two types of fibers, one of which is a superabsorbent fiber. The non-woven web has a graded distribution of fibers, with a resultant gradient in absorbent properties.

It is an object of this invention to provide superabsorbent non-wovens with excellent absorption speed and capacity. It is a further object of this invention to provide superabsorbent non-wovens having excellent conformability, drapability and other fabric-like properties.

According to the present invention, there is provided a non-woven web with a fluid absorption gradient designed to draw and concentrate water away from the source of the water, comprising from about 25% to about 75% superabsorbent fibers and from about 75% to about 25% of at least one other type of fiber, said other fiber having a density differing by at least about 0.2 g/cc from the density of the superabsorbent fibers.

Suitably the web may comprise about 50% superabsorbent fiber.

The invention also provides a process for preparing a non-woven web which comprises:

(a) preparing a 30-60% solids aqueous solution of a polymeric composition which is superabsorbent upon curing;

(b) extruding the solution into fibers;

(c) attenuating the fibers;

(d) collecting the fibers;

(e) chopping the fibers to staple lengths;

(f) blending the fibers with other fibers differing in density by at least 0.2 g/cc;

(g) air-laying the blend to form a non-woven web; and

(h) curing the fibers at any time after extruding to form the graded superabsorbent non-woven web.

In one preferred embodiment the fibers are cured prior to collecting.

Preferably in step (b) the solution is extruded into a column having heated gas flowing therethrough. It is also preferred that the fibers are attenuated by air jets.

One embodiment of this invention provides a layered article with high liquid retention properties comprising at least one layer of a graded non-woven web having from about 25% to 75% hydrophilic fibers and from 75% to 25% other fibers in conjunction with at least one other layer of backing material integrally attached to the non-woven web. optionally the web is needle-punched, adhesively bonded or heat-bonded (thermally fused) to the backing material.

Examples of suitable backing materials are cellulosic, olefinic polyester, acrylic and polyamide films.

The compcsite article may have a first layer of backing material on one face of the non-woven web and a second layer of the same or different backing material on the opposite face of the non-woven web. At least one layer of backing material may be impervious to moisture. At least one layer may be elastic.

In one preferred embodiment, the non-woven web is sandwiched between two layers of cellulosic paper.

The invention further provides an incontinence product, diaper, sanitary napkin or wound dressing having as one element thereof a non-woven web according to the invention. In one embodiment thereof, there is provided an incontinence product comprising a porous film, a layer of non-woven web according to the present invention and an impervious film, with the more highly absorbent side of the web oriented away from the source of the moisture sought to be absorbed.

The superabsorbent fibers are formed from a heat curable aqueous composition of a copolymer and an unreacted compound.

The aqueous composition preferably comprises (a) from about 25 to about 75 mole percent of about 20 to about 80% neutralized carboxylic acid substituted ethylenic units and from about 75 to about 25 mole percent copolymerizable comonomer units, and (b) at least one unreacted compound. The composition will comprise in weight percent from about 90 to about 99 of (a) and about 10 to about 1 of (b). The unreacted compound is selected from compounds which when heated form cross-links or bonds, e.g., hydrogen bonds, between the carboxylic acid pendant units on the copolymer.

Aqueous compositions suitable for forming fibers usable in this invention are taught in the following U.S. patents, the teachings of which are incorporated by reference herein: U.S. 4,788,237; U.S 4,743,244; U.S. 4,705,773; U.S. 4,616,063; U.S.4,813,945. Particularly suitable is an aqueous composition of a partially neutralized isobutylene/maleic anhdyride copolymer and an unreacted compound.

The superabsorbent fibers may be prepared by the dry spinning process disclosed in U.S. 4,731,067 or by the air attenuation process of EP-A-0301804. Alternative fiber-forming methods may be employed as deemed desirable by one skilled in the art. A preferred superabsorbent fiber is Fibersorb (R) SA-7000 fiber, available from Arco Chemical Company, Newtown Square, PA. Fibersorb (R) SA-7000 fiber is spun from an aqueous solution of a partially neutralized isobutylene/maleic anhydride copolymer and an unreacted compound.

In this invention, the superabsorbent fibers are not used alone, but are air-laid into a web with other compatible fibers. It has been found that fibers which have a density differing by about at least 0.2 g/cc from that of the superabsorbent fibers are surprisingly useful in forming an air-laid web. The webs of this invention display a marked gradient in fiber distribution, wherein the superabsorbent fibers are predominantly distributed along one face of the web and the complementary fiber of differing density is predominantly distributed along the other face. In moving from one face to the next, there is, therefore, a gradient of superabsorbent fiber, and an associated gradient of absorbent properties.

In the air-laying process, the fibers are generally processed through a card in order to properly open and intimately mix the fibers. A homogeneous blend becomes an inhomogeneous distribution of staple fiber. In the air-laying machine, the fibers are separated and blown onto a roll with holes. Air passes through the holes and the fibers are deposited on the web. The thickness can be varied continuously.

The fibers with which the superabsorbent fibers are advantageously blended are those which exceed the above-noted density difference of about 0.2 g/cc. The superabsorbent fibers have a density of approximately 1.4 g/cc; therefore, webs having complementary fibers with densities between 1.2 and 1.6 g/cc are not expected to display the marked gradient associated with the adsorbing non-woven webs of this invention. In particular, then, fibers with densities markedly dif ferent from 1.4 g/cc are expected to be of greatest utility in this invention. Fibers which are useful in this invention include, but are not limited to, acrylic, nylon, polyethylene, polypropylene, and poly(vinyl acetate). preferably the complementary fiber is polyolefin, more preferably a polyethylene or a polypropylene staple. Polyester fibers will not yield the desired structure; nor will rayon under most conditions.

One skilled in the art of textile fibers will recognize that fiber denier, finish, crimp, cross-section, length, moisture content, and other factors may affect the final distribution of fibers in the non-woven web. By proper selection of optimum values of these parameters, non-wovens of fibers which are nominally not of this invention due to their non-compliance with the density difference criterion, such as rayon, may exhibit a graded distribution of fibers, thereby becoming equivalents of the preferred fabrics.

The non-woven webs of this invention were tested for their absorbent properties by three tests developed for this purpose. Details of these tests are given below.

Demand Absorption Test

The test procedure is as follows: a piece of hydrophilic paper liner (tea bag paper, approximately 2 square inches) was placed over the opening of the sample platform of a Demand Absorption Tester from Scientific Machine and Supply Co. The level of the burette was then adjusted to bring the brine level up touching the liner and making the liner barely damp. A sample of absorbent (mat, laminate, or powder) approximately one square inch in area and 0.15-0.20 g in weight was placed over the opening on the sample platform on the liner. Fluid wicking started as soon as the burette and air bleed stopcocks were opened. The timer was activated when the first bubbles bled into the burette. The time was recorded at each half milliliter of brine absorbed. The volume of brine absorbed was divided by the initial sample weight to obtain absorption in grams of brine/gram of sample. Plots of demand absorption speeds versus times are used to find the absorption speed (grams of brine/one gram of sample/second) from the initial slope of the curve.

Swell Index

This test procedure is described in U.S. 4,454,055, the teachings of which are incorporated herein by reference thereto. The test procedure and equipment used herein were modified slightly as compared to the procedure and equipment described in U.S. 4,454,055.

To determine the Free Swell Index at atmospheric (room) pressure, about 0.2 to 0.3 g of the water-absorbing composition to be tested is placed in an empty tea bag. The tea bag containing the composition is immersed in brine (0.9 wt. % NaCl) for ten minutes, removed and allowed to sit on a paper towel for thirty seconds to remove surface brine. The Swell Index of the composition, that is, the units of liquid absorbed per each unit of sample is calculated using the following formula:

$$\text{Free Swell Index} = \frac{\text{Weight of Wet Composition}}{\text{Weight of Dry Composition}} - 1$$

To determine Swell Index under pressure, the following modified procedure was used.

After the tea bag containing the sample composition is immersed in brine and surface brine is removed, it is immediately placed in a 16 cm ID Buchner funnel fitted with a 2000 ml sidearm vacuum filter flask and connected to a manometer. Then, a piece of dental dam rubber sheeting is securely fixed over the mouth of the funnel such that the sheeting just rests on the tea bag. Next, a vacuum sufficient to create the desired pressure is drawn on the flask for a period of five minutes, and the Swell Index under pressure is calculated using the above formula.

Example 1

Approximately 3 lbs of Fibersorb (R) SA-7 000 superabsorbent fiber (available from ARCO Chemical company, Newtown Square, PA) were blended with approximately 3 lbs of a 2.2 denier, 1½ inch polypropylene staple (Hercules T-185 - diameter 21 microns). This blend was processed through a Garnett card to open and intimately mix the fibers. The web collected from the Garnett was then fed into a Rando Feeder/Rando Webber processing line and a randomly oriented web made and wrapped on a roll of paper. The width of the web was thirty-six inches with a basis weight of 216 g/m². The web was cut into strips of ten inches width and needle-punched in a James Hunter needling machine. The depth of penetration through the thickness of the web was 3 barbs, and needling intensity was 57 needles/cm². This intensity was achieved in two passes, half from each side of the web.

The web of this example was then subjected to the absorbency tests described above and found to have a Free Swell Index of 26 g/g, a 0.5 psi swell index of 17.5 g/g, and an absorption speed of 0/29 g/g second.

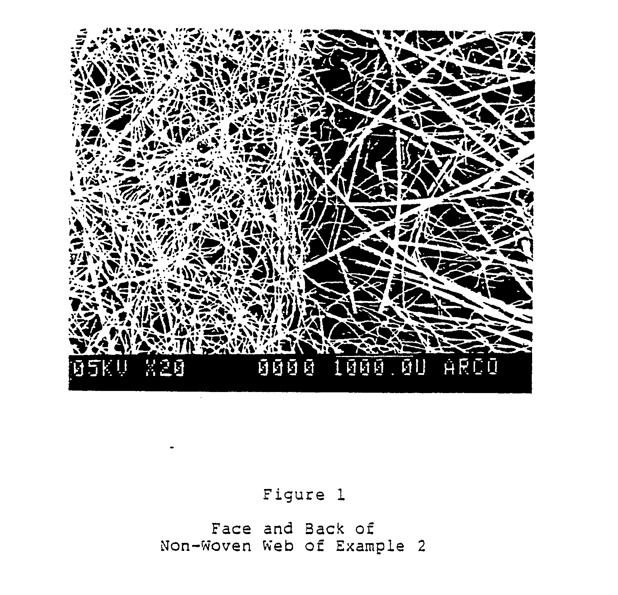

The absorption speed of this blend of polypropylene and Fibersorb (R) super absorbent fiber is unexpected and is believed to be a result of the unique graded distribution of fibers attributable to the high density difference between the polypropylene and the superabsorbent fiber. Fig. 1 shows the face and back of the material produced in this example. It is clear that the superabsorbent fiber distribution is significantly different on the back, as compared to the face. One advantage attributable to this distribution is that there is more open space around the superabsorbent fiber which is apparently desirable for enhancing the swelling properties. The 0.5 psi Swell Index, while lower than the Free Swell Index, indicates that absorbed moisture is not easily removed from the article under pressure.

Comparative Example 2

In a similar manner, 50/50 blends of Fibersorb (R) superabsorbent fiber and 3 denier polyester (18 micron diameter) were prepared and subjected to the above tests. A non-woven web of Fibersorb (R) superabsorbent fiber and polyester fiber is not of this invention since the density difference between these fibers is less than about 0.2.

The results obtained upon subjecting these webs to the absorbency tests were:

| Fiber Blended With Fibersorb(R) Superabsorbent Fiber (50/50 | Density of Other Fiber (g/cc) | Free Swell Index (g/g) | 0.5 psi Swell Index (g/g) | Absorption Speed (g/g/cc) |
|---|---|---|---|---|
| Polypropylene | 0.90 | 27.0 | 17.0 | 0.29 |
| Polyester | 1.38 | 18.5 | ---- | 0.08 |
| Fibersorb(R) (100%) | ---- | 45.0 | 30.0 | 0.28 |

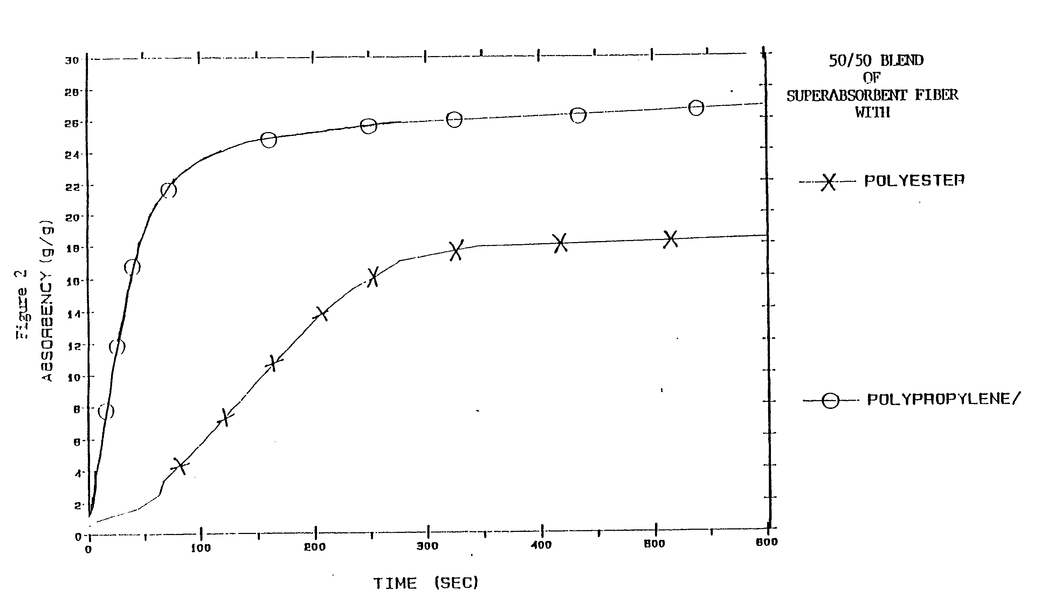

Fig. 2 is a composite plot of the absorption tests summarized in the Table. It is clear that the non-woven webs of this invention have significantly increased rates of absorption as compared to the non-woven web of polyester/superabsorbent fiber. In fact, it appears that the greater the density difference between the superabsorbent fiber and the complementary fiber, the higher the absorption speed. The polypropylene/superabsorbent fiber web exhibits an absorption speed similar to that of a 100% superabsorbent fiber web.

The water-absorbing webs of this invention and articles of manufacture into which the webs are incorporated are suitable for use in a wide range of absorptive functions. In general, the articles into which the water-absorbing webs are incorporated serve the function of supporting the web and presenting it in a form adapted for absorptive end use. Means to support and present the web for absorptive use include, but are not limited to bandages, surgical and dental sponges, tampons, sanitary napkins and pads, disposable diapers, meat trays, pads for absorption of perspiration, and the like.

**Claims**

1. A non-woven web with a fluid absorption gradient designed to draw and concentrate water away from the source of the water, comprising from about 25% to about 75% superabsorbent fibers and from about 75% to about 25% of at least one other type of fiber, said other fiber having a density differing by at least about 0.2 g/cc from the density of the superabsorbent fibers.

2. A non-woven web as claimed in claim 1 in which the concentration of superabsorbent fibers decreases through the thickness of the web from one face to the other.

3. A non-woven web as claimed in claim 1 or claim 2, wherein the superabsorbent fibers are formed from an aqueous composition comprising:

(a) a polymer comprising from about 25 to about 75 mole percent of about 20 to about 80% neutralized carboxylic acid substituted ethylenic units and from about 75 to about 25 mole percent copolymerizable comonomer units, and

(b) at least one unreacted crosslinking compound.

4. A non-woven web as claimed in claim 1, claim 2 or claim 3, wherein the superabsorbent fiber is prepared from an isobutylene/maleic anhydride copolymer

5. A non-woven web as claimed in any one of claims 1 to 4 wherein the superabsorbent fiber is Fibersorb (R) superabsorbent fiber.

6. A non-woven web as claimed in any one of claims 1 to 5 comprising 50% superabsorbent fibers.

7. A non-woven web as claimed in any one of claims 1 to 6 in which the other fibers are polyolefin fibers.

8. A process for preparing a non-woven web which comprises:

(a) preparing a 30-60% solids aqueous solution of a polymeric composition which is superabsorbent upon curing;

(b) extruding the solution into fibers;

(c) attenuating the fibers;

(d) collecting the fibers;

(e) chopping the fibers to staple lengths;

(f) blending the fibers with other fibers differing in density by at least 0.2 g/cc;

(g) air-laying the blend to form a non-woven web; and

(h) curing the fibers after extruding to form the graded superabsorbent non-woven web.

9. A process as claimed in claim 8 in which the step of curing the fibers is effected prior to collecting.

10. A process as claimed in claim 8 or claim 9 in which the solution is extruded into a column having heated gas flowing therethrough.

11. A process as claimed in any one of claims 8 to 10 in which the fibers are attentuated by air jets.

12. A composite article comprising a web as claimed in any one of claims 1 to 7 or obtained by a process as claimed in any one of claims 8 to 11 and at least one layer of a backing material.

13. A composite article of claim 12 in which the non-woven web is thermally fused to the backing material or adhesively bonded to the backing material.

14. A composite article as claimed in claim 12 having a first layer of backing material on one face of the non-woven web and a second layer of the same or different backing material on the opposite face of the non-woven web.

15. A composite article as claimed in claim 14 in which the layers are thermally bonded.

16. A composite article as claimed in claim 14 or claim 15 in which the non-woven web is sandwiched between two layers of cellulosic paper.

17. A composite article as claimed in any one of claims 12 to 16 in which the backing material is a cellulosic, olefinic, polyester, acrylic or polyamide film.

18. A composite article as claimed in any one of claims 12 to 17 in which at least one layer of backing material is impervious to moisture.

19. A composite article as claimed in any one of claims 12 to 18 in which at least one backing material is elastic.

20. An incontinence product comprising a porous film, a layer of a non-woven web as claimed in any one of claims 1 to 7 or obtained by a process as claimed in any one of claims 8 to 11, and an impervious film, in which the more highly absorbent side of the web is oriented away from the source of moisture sought to be absorbed.

21. An incontinence product, diaper, sanitary napkin or wound dressing having as one element a non-woven web as claimed in any one of claims 1 to 7 or obtained by a process as claimed in any one of claims 8 to 11.

22. A non-woven web with a fluid absorption gradient designed to draw and concentrate water away from the source of the water, comprising from about 25% to about 75% superabsorbent fibers and from about 75% to about 25% of at least one other type of fiber, and wherein the concentration of superabsorbent fibers decreases through the thickness of the web from one face to the other.

Figure 1

Face and Back of
Non-Woven Web of Example 2

Figure 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 212 618 (KIMBERLY-CLARK) <br> * the whole document * <br> – – – | 1,2,12, 20-22 | D 04 H 1/70 <br> D 04 H 1/42 <br> A 61 F 13/15 |
| A,D | EP-A-0 272 074 (ARCO) <br> * the whole document * <br> – – – | 1,3,4,22 | |
| A | EP-A-0 301 804 (ARCO) <br> * the whole document * <br> – – – | 8-11,21 | |
| A | US-A-4 102 340 (FREDERICK K.MESEK) <br> * column 1-7; figures 3-4 * <br> – – – | 12-19 | |
| A | EP-A-0 205 242 (KIMBERLY-CLARK) <br> * claims 1-6 * <br> – – – – – | 1,20,22 | |

| | | | TECHNICAL FIELDS SEARCHED (Int. Cl.5) |
|---|---|---|---|
| | | | D 04 H <br> A 61 F <br> A 61 L |

The present search report has been drawn up for all claims

| Place of search | Date of completion of search | Examiner |
|---|---|---|
| The Hague | 27 November 90 | DURAND F.C. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention

E : earlier patent document, but published on, or after the filing date
D : document cited in the application
L : document cited for other reasons

................................................................

& : member of the same patent family, corresponding document